# EUROPEAN PATENT APPLICATION

(11) **EP 4 243 028 A1**
(43) Date of publication of application: **13.09.2023**
(21) Application number: 22382229.7
(22) Date of filing: 10.03.2022
(51) Int. Cl.: G16H 10/40, G16H 40/20, G16H 40/40

(54) **INTELLIGENT USER GUIDANCE FOR LABORATORY ISSUE RESOLUTION**

(71) Applicant: F. Hoffmann-La Roche AG, 4070 Basel (CH); Roche Diagnostics GmbH, 68305 Mannheim (DE)
(72) Inventor: HOFSTETTER, Hermann, 6343 Rotkreuz (CH); MORCILLO MONTEJO, Alejandro, 08174 Sant Cugat del Vallès - Barcelona (ES)
(74) Representative: Herren, Barbara

(57) **Abstract**

A computer-implemented method of providing user guidance for laboratory issue resolution within a laboratory system is presented. The method comprises monitoring activity by a user within a laboratory setting of the laboratory system by a user guidance system, detecting an laboratory issue within the laboratory system, retrieving by the user guidance system a preferred resolution to the detected laboratory issue and typical time to perform preferred solution from a database, notifying the user to the laboratory issue, detecting a non-preferred issue resolution for the laboratory issue being performed by the user, displaying the user guidance system to the user, wherein the user guidance system provides the retrieved preferred resolution for the laboratory issue to the user, resolving the laboratory issue by the user using the user guidance system, and removing the display of the user guidance system following the resolution of the laboratory issue.

## Description

### Technical Field

The present disclosure generally relates to providing minimally intrusive guidance to a laboratory user for laboratory issue resolution within a laboratory system.

### Background

Current laboratory systems are increasingly becoming more complex as they progress towards more fully automated models. As these laboratory systems become more complex, a burden can be created on laboratory workers as they are increasingly faced with multiple laboratory instruments and IT laboratory solutions that each have distinct protocols and manuals and require highly specialized operator skill sets.

Document US Pat. 9,407,786 discloses methods and systems designed to facilitate troubleshooting and maintenance of in-vitro diagnostic equipment via an app on a mobile device. The app receives status information and images of the equipment and uses this data to provide tutorials and interactive remote technical support to a user.

Document EP 2299277 B discloses informing a user upon logging into a server about one or more steps of a workflow that need to be executed by that user on at least a first or second lab device.

Document US Pat. 6,834,207 discloses an operating guidance system for a medical system whereby it is possible to give instruction on the operation of a medical imaging system remotely.

### Summary

It is an object of the present disclosure to provide an efficient support for laboratory users to resolve laboratory issues while simultaneously being as minimally intrusive so that laboratory work disturbance is kept at a minimum.

According to a first aspect of the present discloser, a computer-implemented method of providing user guidance for laboratory issue resolution within a laboratory system is presented. The method comprises monitoring activity by a laboratory user within a laboratory setting of the laboratory system by a user guidance system, detecting an laboratory issue within the laboratory system, retrieving by the user guidance system a preferred resolution to the detected laboratory issue and typical time to perform preferred solution from a database, notifying the laboratory user to the laboratory issue, detecting a non-preferred issue resolution for the laboratory issue being performed by the laboratory user, displaying the user guidance system to the laboratory user, wherein the user guidance system provides the retrieved preferred laboratory issue resolution for the laboratory issue to the laboratory user, resolving the laboratory issue by the laboratory user using the user guidance system, and removing the display of the user guidance system following the resolution of the laboratory issue.

The activity by the laboratory user can be interactions with laboratory management systems, interactions with information technology (IT) systems, interactions with laboratory instruments, and/or combinations thereof.

The laboratory issue can comprise technical issues, patient security issues, worker security issues, laboratory instrument issues, software maintenance issues, and/or combinations thereof.

The computer-implemented method can further comprise, if the detected non-preferred issue resolution for the laboratory issue is better, i.e., more optimal than the retrieved preferred issue resolution, saving the detected non-preferred issue resolution in the database for possible future guidance use. Examples of better/more optimal issue resolution can be issue resolutions that result in fewer method steps, fewer laboratory system disturbances, less reagent waste, less test sample waste, quicker resolution execution, and/or combination thereof.

The computer-implemented method can further comprise determining the skill level of the laboratory user by the user guidance system based on previous guidance sessions of the laboratory user with the user guidance system. If the skill level of the laboratory user is determined to be low, requesting additional training to be given to that laboratory user and monitoring adherence and effectiveness of the laboratory user to the additional training by the user guidance system.

The computer-implemented method can further comprise providing a ranked listing of the additional training provided by the user guidance system to the laboratory user based on the adherence and effectiveness of the laboratory user.

The computer-implemented method can further comprise, before removal of the display of the user guidance system, requesting feedback from the laboratory user regarding the quality of the guidance provided by the user guidance system to the laboratory user and uploading the laboratory user feedback to the database.

The computer-implemented method can further comprise providing a ranked listing of preferred issue resolutions provided by the user guidance system. The ranking of the preferred issue resolutions can be based on the uploaded laboratory user feedback, required numbers of method steps to perform the issue resolution, least number of laboratory system interruptions, and/or least amount of reagents and/or test samples wasted.

The computer-implemented method can further comprise contacting a field service representative of the laboratory system if the laboratory issue cannot be resolved by the laboratory user using the user guidance system.

The computer-implemented method can further comprise detecting laboratory issues without preferred laboratory issue resolutions stored in the database, tracking laboratory user interactions performed by the laboratory user in order to resolve the detected new laboratory issues, and storing the new laboratory resolutions to the detected new laboratory issues in the database for future guidance use.

The computer-implemented method can further comprise providing the user guidance system to a field service representative to support in the initial installation and configuration of the laboratory system.

The user guidance system can be displayed on an output display of the laboratory system to the laboratory user.

The computer-implemented method can further comprise allowing the user guidance system to resolve the laboratory issue without input from the laboratory user.

There are several advantages to the present disclosure. Namely, there will be a decrease of cost for support and training for laboratory users in a laboratory environment. The user guidance system can provide "on the job training" and support without the reliance of field service representative (FSR) of the laboratory system having to be present on-site to provide such services.

Additionally, the user guidance system can result in higher user satisfaction due to laboratory issue resolution and support in real time at any time, which can result in increased laboratory user task proficiency and laboratory system knowledge.

In addition, the presence of a non-intrusive user guidance system that does not disturb the normal work pace of a laboratory and that is only visible upon detection of a laboratory user need is highly attraction in a laboratory setting.

Further, through the use and feedback of the analytic metrics engine of the intelligent user guidance system, the non-intrusive system will be able to capture laboratory user interactions with the laboratory system, which in turn, can improve the user guidance system of the laboratory system and/or help identify problematic areas of the laboratory system.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a block diagram of the intelligent user guidance system according to an embodiment of the present disclosure.
Fig. 2 illustrates a laboratory user use case of the intelligent user guidance system according to an embodiment of the present disclosure.
Fig. 3 illustrates a flowchart of the intelligent user guidance system according to an embodiment of the present disclosure.
Fig. 4 illustrates a flowchart of the intelligent user guidance and training system according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

The use of the 'a' or 'an' can be employed to describe elements and components of the embodiments herein. This is done merely for convenience and to give a general sense of the inventive concepts. This description should be read to include one or at least one and the singular includes the plural unless it is obvious that it is meant otherwise.

The term 'laboratory instrument' or "laboratory device" as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis and the like. The term 'instrument' or 'device' can cover pre-analytical instruments/devices, post-analytical instruments/devices, analytical instruments/devices and laboratory middleware.

A 'data storage unit' or 'database' can be a computing unit for storing and managing data such as a memory, hard disk or cloud storage. This may involve data relating to biological/medical test sample(s) to be processed by the automated system. The data management unit may be connected to an LIS (laboratory information system) and/or an HIS (hospital information system). The data management unit can be a unit within or co-located with a laboratory instrument/device. It may be part of the laboratory middleware. Alternatively, the database may be a unit remotely located. For instance, it may be embodied in a computer connected via a communication network.

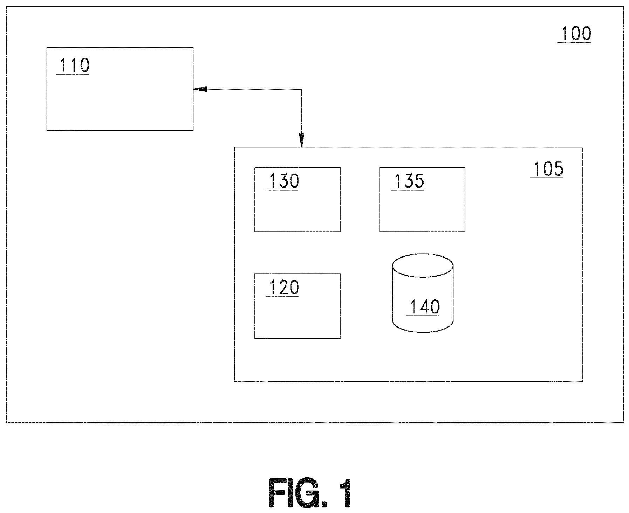

Referring initially to Fig. 1, a simple block diagram of an intelligent user guidance system 100 for a laboratory system is illustrated. The intelligent user guidance system 100 can comprise a non-intrusive user interface 110 to the laboratory system and a controller 105 comprising a user behavior observation system 120, an intelligent user guidance analytics system 130, an analytic metrics engine 135, and an intelligent user guidance database 140. In one embodiment, the intelligent user guidance system 100 can be resident in the laboratory system. In another embodiment, the intelligent user guidance system 100 can be located remotely from the laboratory system. The non-intrusive user interface 110 can be part of the general user interface of the laboratory system in which the laboratory user typically interacts with the laboratory system such as, for example, an output display screen.

A laboratory user can be any person that performs typical laboratory activities, or tasks, in a laboratory system setting such as, for example, a laboratory technician. Such typical laboratory tasks can be monitoring the laboratory system for laboratory issues, performing service/maintenance on the laboratory system, and/or refilling reagents and/or other laboratory consumables.

The intelligent user guidance database 140 can comprise a set of known laboratory issues/problems along with the optimum solutions/mitigations for those known laboratory issues/problems. The intelligent user guidance database 140 can also maintain laboratory user feedback associated with the optimum solutions/mitigations. Additionally, the intelligent user guidance database 140 can also comprise and maintain a list of known laboratory users in the laboratory system as well as the associated laboratory system training the laboratory users have completed.

The intelligent user guidance system 100 can survey/observe laboratory user activity in a non-intrusion fashion as the laboratory user attempts to solve a laboratory issue occurring in the laboratory system. The term "non-intrusive" can refer to a program or interface that is not visible to a laboratory user as the laboratory user performs his/her laboratory tasks and that does not hinder the laboratory user as the laboratory user performs the laboratory tasks.

The user activity can comprise interactions with the information technology (IT) systems managing the laboratory system or the IT system itself or a laboratory instrument such as, for example, a laboratory analyzer.

The laboratory issue can be any laboratory issue/problem that might occur in the laboratory system that may affect the performance of the laboratory system such as, for example, patient security, worker security, laboratory instrument issues, software maintenance tasks, and the like.

Upon detection of a non-optimal course of action by the laboratory user to resolve the particular laboratory issue, the intelligent user guidance system will trigger the appearance of the intelligent user guidance system on the user interface the laboratory user is using. The intelligent user guidance system can then provide an optimum approach to the laboratory user in order to resolve/mitigate the laboratory issue.

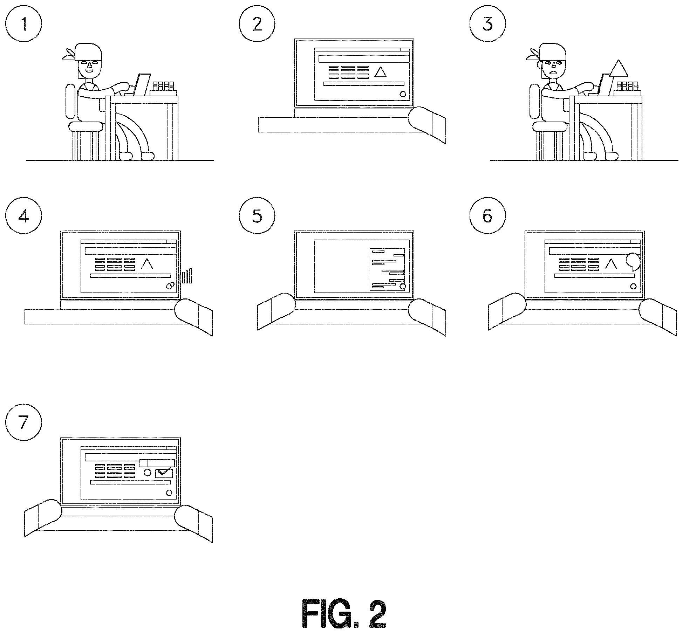

Fig. 2 illustrates a laboratory user use case of the intelligent user guidance system. In Box 1 of the use case, a laboratory user is performing tasks on a laboratory system. In Box 2 of the use case, the laboratory system notifies the laboratory user that there is a laboratory issue with the laboratory system that needs the attention of the laboratory user by generating an alarm, an alert and/or a warning display (e.g., a warning triangle on the laboratory user display). In Box 3 of the use case, the laboratory user begins to attempt to resolve the laboratory issue. In Box 4 of the use case, the intelligent user guidance system determines the laboratory issue and matches the optimal solution in the user guidance database to that laboratory issue that the laboratory user is trying to resolve. In Box 5 of the use case, the intelligent user guidance system appears on the laboratory user's display and notifies the laboratory user that there is a better/more optimal approach to resolving the laboratory issue and asks the laboratory user if the laboratory user would like help resolving the laboratory issue. In Box 6 of the use case, the intelligent user guidance system guides the laboratory user through the more optimal laboratory issue resolution, if the laboratory user accepts the help offered in Box 5 to solve the laboratory issue, while the laboratory user still maintains control of the laboratory system. In Box 7 of the use case, the laboratory issue has been resolved and the laboratory user has gained knowledge on how to resolve the laboratory issue the next time the laboratory issue is encountered by the laboratory user in the laboratory system.

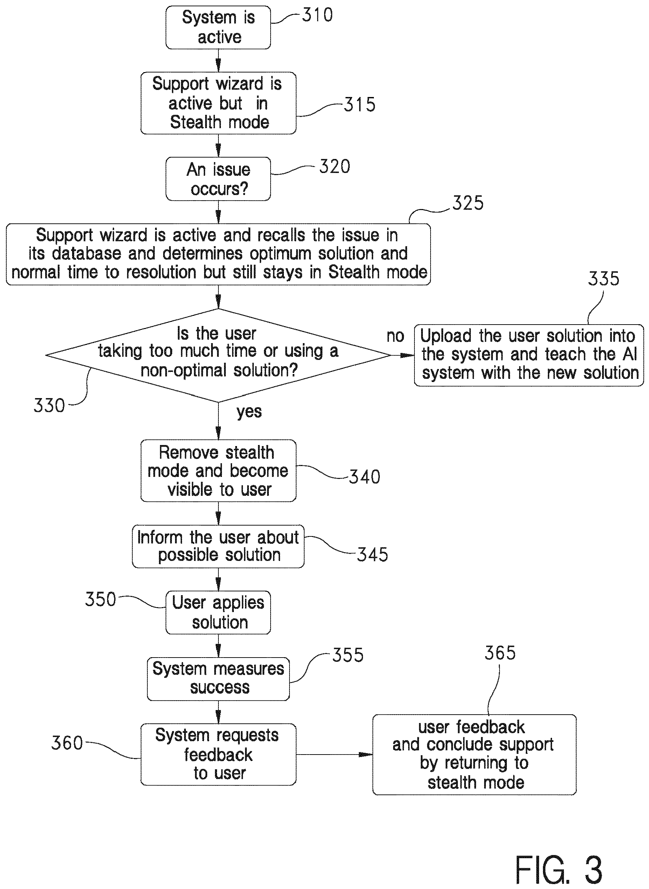

Fig. 3 illustrates a flowchart of the method of intelligent user guidance system. According to Fig. 3, upon the starting of the laboratory system, step 310, the intelligent user guidance system is also started, step 315, but remains in stealth mode, i.e., not visible on the user interface to the laboratory user.

When a laboratory issue occurs and is detected by the intelligent user guidance system, in step 320, the intelligent user guidance system will ping and retrieve from an intelligent user guidance database a preferred possible solution for the laboratory issue as well as a preferred time of resolution for that laboratory issue, in step 325. A laboratory issue can comprise a laboratory system generated task or message with a warning and associated error severity such as, for example, reagent is running low or an error that a test sample has a clot and test result cannot be shown. The intelligent user guidance system can then detect that the laboratory user is interacting with the user interface of the laboratory system and that the laboratory user is not exhibiting proper action to solve the laboratory issue and/or is not acknowledging the laboratory system error message/warning of the laboratory issue.

In step 330, the intelligent user guidance system will remain in stealth mode and will start a timer for laboratory issue resolution. At the same time, the intelligent user guidance system will assess the laboratory issue resolution actions of the laboratory user and compare these actions of the laboratory user to the preferred laboratory issue resolution. In the event the laboratory user exceeds the preferred time of resolution or deviates from the preferred laboratory issue resolution, the intelligent user guidance system will exit stealth mode and will become visible to the laboratory user, in step 340, on the laboratory user interface of the laboratory system. At this point, the intelligent user guidance system will inform the laboratory user of the preferred laboratory issue resolution, in step 345, and ask the laboratory user if s/he would like guidance in the resolution of the laboratory issue..

It is possible that more than one laboratory issue solution/resolution/mitigation for the laboratory issue is available in the intelligent user guidance database. In this case, the laboratory issue resolutions can be ranked by the analytic metrics engine 135 of the intelligent user guidance system before being presented to the laboratory user. The rankings of laboratory issue resolutions can be based on different laboratory issue resolution metrics such as, for example, the number of steps by the laboratory user that are required for laboratory issue resolution, the fewest laboratory system interruptions, and/or the least amount of wasting of reagents or test samples.

Upon resolution of the laboratory issue, in step 350, by the laboratory user following instructions/guidance to the preferred laboratory issue resolution from the intelligent user guidance system, the intelligent user guidance system can, then, measure success of instructions, i.e., the laboratory issue elimination or mitigation, in step 355, by the analytic metrics engine 135, i.e., how well did the laboratory user follow the instructions/guidance provided the intelligent user guidance system.

The intelligent user guidance system will then return to stealth mode after resolution of the laboratory issue. In one embodiment, the instructions given by the intelligent user guidance system can be given by visual indications on a display of the user interface in which the laboratory user performs the resolution steps for the laboratory issue. In another embodiment, the intelligent user guidance system can be given control of the laboratory user interface by the laboratory user and the intelligent user guidance system can then resolve the laboratory issue for the laboratory user. At any time during this embodiment, however, the laboratory user can regain control of the user interface from the intelligent user guidance system and, at which point, the intelligent user guidance system will return to stealth mode, i.e., will no longer be visible to the laboratory user.

At the resolution of the laboratory issue and/or at the time the intelligent user guidance system returns to the stealth mode, the analytic metrics engine 135 of the intelligent user guidance system can request feedback, in step 360, from the laboratory user as to the effectiveness/ease of the instructions/guidance received by the intelligent user guidance system. This laboratory user feedback can then be uploaded to the intelligent user guidance database 140 in step 365, and stored with the laboratory issue resolution. This laboratory user feedback can then be used to rank, or score, the laboratory issue resolutions presented to the other laboratory users in the future.

In addition, or alternatively, the intelligent user guidance system may determine that the laboratory issue resolution activity of the laboratory user may be better/more optimal than the laboratory issue resolution currently mapped to the laboratory issue in the intelligent user guidance database 140. For example, the laboratory user may be resolving/mitigating the laboratory issue faster than the currently mapped resolution. In this case, the analytic metrics engine 135 of the intelligent user guidance system may uploaded the laboratory user's laboratory issue resolution to the intelligent user guidance database 140 as another possible resolution to the laboratory issue after verification that this laboratory issue resolution is indeed better/more optimal than the currently mapped laboratory issue resolution in step 335. This laboratory issue resolution then may be presented as alternative laboratory issue resolution/mitigation to the laboratory issue to other laboratory users in the future.

The different methods the laboratory issue resolution may be considered better than the currently mapped laboratory issue resolution can include, for example, requiring fewer steps to resolution, requiring less shutdown time by the laboratory system, less waste of reagents or other consumables, less re-extraction of test samples, less time than average tracked for laboratory user interactions for that specific laboratory issue, and the like.

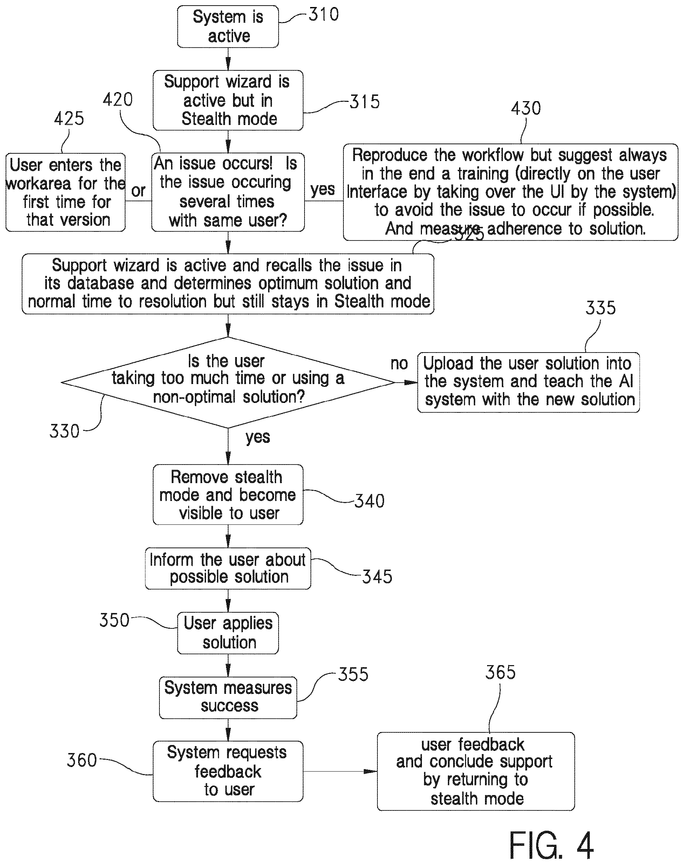

Fig. 4 illustrates a flowchart of the method of intelligent user guidance and training system. In this embodiment, the intelligent user guidance system can identify the laboratory user of the laboratory system from the list stored on user guidance database 140 and determine the level of expertise the laboratory user possess such as, for example, can determine if the laboratory user is a first time user of the laboratory system, step 425, or a laboratory user who has consistently required assistance for this particular laboratory issue, in step 430. In this embodiment, the intelligent user guidance system will provide the same guidance as disclosed in Fig. 3 but, at the time of laboratory issue occurrence, at step 420, the intelligent user guidance system can identify the laboratory user. In addition, or alternatively, at the end of the laboratory issue resolution, at step 350, additional training may be suggested for that laboratory user.

The analytic metrics engine 135 of the intelligent user guidance system, in this embodiment, can measure not only laboratory user adherence to the training but also the effectiveness/impact of the training, i.e., the intelligent user guidance system can measure if the guidance provided by the intelligent user guidance system had a positive impact on the laboratory user performance during the resolution of the laboratory issue.

The analytic metrics engine 135 of the intelligent user guidance system may also obtain further detailed metrics of the particular impact of the intelligent user guidance system across the overall laboratory issue solving/mitigation tasks and can provide a ranked list of the training according to the training's impact in laboratory issue resolution so that laboratory users perform, at least, the most important or most critical trainings.

In one embodiment, if it is determined that the laboratory user and/or the intelligent user guidance system is unable to resolve the laboratory issue, the intelligent user guidance system can notify a field service representative (FSR) of the laboratory system via, for example, a communication network and schedule an appointment for the FSR to come and service the laboratory system.

In one embodiment, the intelligent user guidance system may also be used during the initial installation of the laboratory system by a FSR. The intelligent user guidance system can support and lead the FSR during the initial configuration. In this way, the use of the intelligent user guidance system can help reduce the overall burden of the FSR during very complex laboratory system installations.

Additionally, the analytic metrics engine 135 of the intelligent user guidance system can also be used to detect new laboratory issues not currently in the intelligent user guidance database by monitoring and measuring laboratory user interactions with the laboratory system. For example, if the laboratory user is working in a laboratory workflow that currently has no intelligent user guidance system content in the intelligent user guidance database and an laboratory error or warning occurs in this laboratory workflow in which the laboratory user needs to interact with the laboratory system, the intelligent user guidance system can activate a tracking mode and log the laboratory user interactions with the laboratory system until the error or warning issue is resolved. This tracking will be stored in the intelligent user guidance database as a resolution to that laboratory issue and can be used for guidance to resolve any future occurrences of this laboratory issue.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a paper format, or on a computer-readable data carrier on premise or located at a remote location. Specifically, the computer program product may be distributed over a data network (such as a cloud environment). Furthermore, not only the computer program product, but also the execution hardware may be located on premise or in a cloud environment.

Further disclosed and proposed is a computer-readable medium comprising instructions which, when executed by a computer system, cause a laboratory automation system to perform the method according to one or more of the embodiments disclosed herein.

Further disclosed and proposed is a modulated data signal comprising instructions, which, when executed by a computer system, cause a laboratory automation system to perform the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A computer-implemented method of providing user guidance for laboratory issue resolution within a laboratory system, the method comprising:
monitoring activity by a laboratory user within a laboratory setting of the laboratory system by a user guidance system 100;
detecting an laboratory issue within the laboratory system;
retrieving by the user guidance system 100 a preferred resolution to the detected laboratory issue and typical time to perform preferred solution from a database 140;
notifying the laboratory user to the laboratory issue;
detecting a non-preferred issue resolution for the laboratory issue being performed by the laboratory user;
displaying the user guidance system 100 to the laboratory user, wherein the user guidance system 100 provides the retrieved preferred resolution for the laboratory issue to the laboratory user;
resolving the laboratory issue by the laboratory user using the user guidance system 100; and
removing the display of the user guidance system 100 following the resolution of the laboratory issue.

2. The computer-implemented method according to claim 1, wherein the activity by the laboratory user is interactions with laboratory management systems, interactions with information technology systems, interactions with laboratory instruments, and/or combinations thereof.

3. The computer-implemented method according to claim 1, wherein the laboratory issue comprises technical issues, patient security issues, worker security issues, laboratory instrument issues, software maintenance issues, and/or combinations thereof.

4. The computer-implemented method according to claim 1, further comprising,
if the detected non-preferred issue resolution for the laboratory issue is more optimal than the retrieved preferred issue resolution, saving the detected non-preferred issue resolution in the database 140 for possible future guidance.

5. The computer-implemented method according to claim 4, wherein a more optimal issue resolution comprises fewer method steps, fewer laboratory system disturbances, less reagent waste, less sample waste, quicker resolution execution, and/or combination thereof.

6. The computer-implemented method according to claim 1, further comprising,
determining skill level of the laboratory user based on previous guidance sessions of the laboratory user with the user guidance system 100;
if the skill level of the laboratory user is determined to be low, requesting additional training be given to that laboratory user; and
monitoring adherence and effectiveness of the laboratory user to the additional training by the user guidance system 100.

7. The computer-implemented method according to claim 6, further comprising,
providing a ranked listing of the additional training provided by the user guidance system 100 to the laboratory user based on the adherence and effectiveness of the laboratory user.

8. The computer-implemented method according to claim 1, further comprising,
before removal of the display of the user guidance system 100, requesting feedback from the laboratory user regarding quality of guidance provided by the user guidance system 100; and
uploading user feedback to the database 140.

9. The computer-implemented method according to claim 1, further comprising,
providing a ranked listing of preferred issue resolutions provided by the user guidance system 100.

10. The computer-implemented method according to claim 9, wherein ranking of the preferred resolutions is based on laboratory user feedback, required numbers of method steps to perform the preferred issue resolution, least number of laboratory system interruptions, and/or least amount of reagents and/or test samples wasted.

11. The computer-implemented method according to claim 1, further comprising,
contacting a service representative of the laboratory system if the laboratory issue cannot be resolved by the laboratory user using the user guidance system 100.

12. The computer-implemented method according to claim 1, further comprising,
detecting laboratory issues without preferred resolutions stored in the database 140; and
tracking laboratory user interactions performed by the laboratory user in order to resolve detected new laboratory issues; and
storing laboratory resolutions to the detected new laboratory issues in the database 140 for future guidance.

13. The computer-implemented method according to claim 1, further comprising,
providing the user guidance system 100 to a field service representative of the laboratory system to support in the initial installation and configuration of the laboratory system.

14. The computer-implemented method according to claim 1, wherein the user guidance system 100 is displayed on an output display of the laboratory system to the laboratory user.

15. The computer-implemented method according to claim 1, further comprising,
allowing the user guidance system 100 to resolve the laboratory issue without input from the laboratory user.
